# EUROPÄISCHE PATENTSCHRIFT

(11) **EP 1 169 267 B1**
(45) Veröffentlichungstag und Bekanntmachung des Hinweises auf die Patenterteilung: **14.07.2004**
(21) Anmeldenummer: 00920394.4
(22) Anmeldetag: 16.03.2000
(51) Int. Cl.: C02F 1/32

(54) **VORRICHTUNG ZUR UV-BESTRAHLUNG VON FLÜSSIGKEITEN**
DEVICE FOR THE UV RADIATION OF LIQUIDS
DISPOSITIF D'EXPOSITION DE LIQUIDES AUX RAYONS U.V.

(30) Priorität: 17.03.1999 US 271090
(43) Veröffentlichungstag der Anmeldung: 09.01.2002
(73) Patentinhaber: WEDECO AG Water Technology, 40472 Düsseldorf (DE)
(72) Erfinder: WEDEKAMP, Horst, D-32052 Herford (DE)
(74) Vertreter: Patentanwälte Thömen & Körner
(86) Internationale Anmeldenummer: PCT/DE2000/000793
(87) Internationale Veröffentlichungsnummer: WO 2000/055095

(56) Entgegenhaltungen:
- EP-A- 0 811 579
- DE-A- 4 206 596
- US-A- 5 780 860

## Beschreibung

Die Erfindung betrifft eine Vorrichtung zur UV-Bestrahlung von Flüssigkeiten.

Solche Vorrichtungen werden vorzugsweise bei Kläranlagen benötigt. Bevor das aus der Kläranlage kommende und gereinigte (bzw. vorgereinigte) Abwasser in natürliche Gewässer eingeleitet wird, muß es immer öfter desinfiziert werden. Zu diesem Zweck wird das durch die Kläranlage gereinigte Abwasser einer ultravioletten Bestrahlung (UV-Bestrahlung) ausgesetzt.

Die dafür vorgesehenen Vorrichtungen besitzen eine Bestrahlungskammer, durch welche das Abwasser fließt. In der Bestrahlungskammer befinden sich UV-Strahlungsquellen, die durch UV-Strahlerlampen gebildet sind, welche zum Schutz gegen das Abwasser von Schutzrohren aus Quarz umhüllt sind. Diese Schutzrohre sind durchlässig für die UV-Strahlen, welche somit in das Abwasser gelangen können.

Neben der Bestrahlungskammer umfassen die bekannten Vorrichtungen eine sich an die Bestrahlungskammer anschließende Auslaufkammer, in welche das der UV-Strahlung ausgesetzte Abwasser gelangt, und von der Auslaufkammer wird das nunmehr gereinigte desinfizierte Abwasser in das Ableitungssystem abgeben.

Durch das Dokument EP 0 687 201 B1 ist beispielsweise eine entsprechende Vorrichtung bekannt, wobei die Bestrahlungskammer als eine vollständig geschlossene Kammer nach Art eines Rohres ausgebildet ist. Durch diese Bestrahlungskammer wird das Abwasser mit einem Druck gepreßt, was zu einer erhöhten Strömungsgeschwindigkeit des Abwassers in der Bestrahlungskammer führt.

Das Dokument DE 42 06 596 A1 beschreibt eine UV-Desinfektionsvorrichtung zur Behandlung von Flüssigkeiten mit einer Bestrahlungskammer, durch welche die Flüssigkeit fließt und in welcher die UV-Strahlungsquellen angeordnet sind, und einer sich in Strömungsrichtung der Flüssigkeit an die Bestrahlungskammer anschließenden und zum Abfluß der Flüssigkeit vorgesehenen Auslaufkammer. Die Auslaufkammer ist als oben offenes Gerinne mit einem unteren Gerinnenboden und zwei Seitenwänden ausgebildet und besitzt einen Wehrkörper, der eine die Strömung der Flüssigkeit abbremsende und den hinteren Abschluß der Auslaufkammer bildende Stauwand mit oberen Überlaufkanten aufweist, über welche die aufgestaute Flüssigkeit überläuft und abfließt. Die Stauwand streckt sich in Strömungsrichtung als nach oben hochgezogene Verlängerung des Gerinnenbodens schräg zum hinteren Ende der Auslaufkammer in einem flachen Winkel.

Bei solchen Vorrichtungen besitzt die sich an die Bestrahlungskammer anschließende Auslaufkammer als Wehrkörper dienende Einbauten in Form von Abschlußwänden, die z.B. als Absperrklappen, Überlaufwehre oder Motorwehre ausgebildet sind, und die sich senkrecht zur Strömungsrichtung des fließenden Abwassers erstrecken. Vor diesen Einbauten staut sich das Wasser und fließt dann über die sogenannten Überlaufkanten, also über die oberen Begrenzungen oder unterhalb der Absperrklappen in das Ableitungssystem ab und wird dem natürlichen Gewässer zugeführt.

Bei bekannten Vorrichtungen umfassen die als Wehrkörper dienenden Einbauten der Auslaufkammer mehrere nebeneinander liegende rechteckförmige oder auch dreieckförmige einzelne Kammern mit senkrechten als Stauwände dienenden Abschlußwänden, wodurch insgesamt die Länge der Überlaufkante für das Abwasser erhöht wird. Somit kann auch relativ viel Abwasser ohne wesentliche Erhöhung des Wasserstandes über die Überlaufkanten ablaufen und an das Ablaufsystem abgegeben werden.

Da bei den bekannten Vorrichtungen zur Vermeidung starker Wasserstands-Schwankungen eine große Länge der insgesamt zur Verfügung stehenden Überlaufkante benötigt wird, sind die Abmessungen der Kammern in Strömungsrichtung des Abwassers gesehen sehr groß, so daß auch insgesamt die komplette Vorrichtung sehr viel Platz beansprucht. Dies führt weiterhin zu einem erhöhten Aufwand an Herstellungskosten und Betriebskosten.

Ein weiterer Nachteil besteht darin, daß das von der Bestrahlungskammer in die Auslaufkammer gelangende Abwasser auf quasi senkrechte Abschlußwände der Einbauten in der Auslaufkammer aufprallt und dort abgebremst wird. Dadurch bildet sich ein Rückstau, der sich bis in die vor der Auslaufkammer liegende Bestrahlungskammer erstrecken kann und in der Bestrahlungskammer zu einer ungleichmäßigen Strömung des Abwassers führt. Eine solche ungleichmäßige Strömung ist aber nachteilig für die Behandlung des Abwassers durch die UV-Strahlen. Es ist zwar möglich, zwischen der Bestrahlungskammer und der Auslaufkammer einen freien Raum in Form einer Beruhigungszone für das fließende Abwasser vorzusehen, um zu verhindern, daß sich der Rückstau bis in die Bestrahlungskammer erstreckt, allerdings werden durch eine solche Beruhigungszone die gesamten Abmessungen der Vorrichtung noch weiter vergrößert, so daß die Kosten einer entsprechenden Vorrichtung nochmals erhöht werden.

Der Erfindung liegt die Aufgabe zugrunde, eine Vorrichtung zur UV-Bestrahlung von Flüssigkeiten, insbesondere von vorgereinigtem Abwasser, zu schaffen, welche unter Beibehaltung einer optimalen Desinfektion und Behandlung von Flüssigkeiten wesentlich kostengünstiger und platzsparender aufgebaut werden kann. Dabei soll es ohne Bedeutung sein, ob die Bestrahlungskammer als eine allseits geschlossene Kammer oder in an sich bekannter Weise als eine Kammer realisiert ist, die durch ein nach oben offenes Gerinne mit einem Boden und zwei Seitenwänden ausgebildet ist.

Die Lösung dieser Aufgabe erfolgt bei der im Oberbegriff des Patentanspruchs 1 vorausgesetzten Vorrichtung durch die Merkmale des kennzeichnenden Teils des Patentanspruchs 1.

Bei der Erfindung erstreckt sich die Stauwand bzw. die Abschlußwand, auf welche das strömende Abwasser trifft, als nach oben hochgezogene Verlängerung des Gerinnebodens schräg vom vorderen Ende zum hinteren Ende der Auslaufkammer in einem flachen Winkel von kleiner als 60° zum Gerinneboden. Das strömende Abwasser trifft also nicht mehr auf eine senkrechte Stauwand. Vielmehr wird das Abwasser durch die schräge Stauwand nur langsam abgebremst, so daß sich die Strömungsgeschwindigkeit nur geringfügig ändert. Das bedeutet, daß kein bzw. ein nur geringer Rückstau der Flüssigkeit eintritt und daß somit die in der Bestrahlungskammer angestrebte gleichmäßige Strömung der Flüssigkeit nicht oder nur unwesentlich beeinflußt wird. Auf eine Beruhigungszone zwischen der Bestrahlungskammer und der Auslaufkammer kann somit bei der Erfindung verzichtet werden, wodurch sich die Kosten der erfindungsgemäßen Vorrichtung reduzieren lassen. Auch der gesamte Platzbedarf der erfindungsgemäßen Vorrichtung läßt sich verringern.

Bei der Erfindung sind weiterhin auf der schrägen Stauwand mehrere voneinander getrennte einzelne Wehrkörper angeordnet. Jeder Wehrkörper ist durch ein sich nach oben erstreckendes hohles und an beiden Enden offenes Rohr gebildet. Die oberen Enden der Rohre bilden die Überlaufkanten zum Überlauf und zum Abfluß des Abwassers. An den Stellen, an denen die Rohre auf der Stauwand befestigt sind, ist die Stauwand mit den Querschnitten der Rohre entsprechenden Öffnungen zum Abfluß der über die Überlaufkanten der Rohre fließenden Flüssigkeit versehen.

Jedes Rohr besitzt an seinem oberen Ende Überlaufkanten, welche durch den Umfang des Rohres vorgegeben sind. Da eine Vielzahl von Rohren vorgesehen ist, ergibt sich insgesamt im Hinblick auf die Überlaufkanten eine große Länge der insgesamt für die Flüssigkeit zur Verfügung stehenden Überlaufkante, so daß die Vorrichtung auch bei einer großen Menge von anfallendem Abwasser mit relativ geringen Wasserstands-Schwankungen zufriedenstellend arbeiten kann und sich die Abmessungen der Auslaufkammer in Längsrichtung gering halten lassen. Wegen der schräg angeordneten Stauwand wird die Strömung der Flüssigkeit nur langsam abgebremst, so daß die Flüssigkeit ohne Bildung eines Rückstaus beruhigt ansteigen kann. Bei erhöhtem Anfall von Flüssigkeit kann die zusätzlich angestaute Menge des Abwassers im übrigen ohne Probleme über die durch die Vielzahl der Rohre gebildeten Überlaufkanten abfließen. Die Strömung in der Bestrahlungskammer bleibt dabei in gewünschter Weise gleichmäßig erhalten, so daß die UV-Bestrahlung der Flüssigkeit in optimaler Weise gewährleistet ist.

In zweckmäßiger Ausgestaltung der Erfindung ist vorgesehen, daß die schräge Stauwand in einem flachen Winkel zwischen 10°- 50° zum Gerinneboden des offenen Gerinnes verläuft. In praktischen Versuchen hat sich gezeigt, daß bei diesen Bereichen eine wirkungsvolle Arbeitsweise der erfindungsgemäßen Vorrichtung gewährleistet ist.

Eine andere Ausgestaltung der Erfindung sieht vor, daß die schräge Stauwand innerhalb des Winkelbereiches zwischen 10° und 50° verschwenkbar und auf dazwischen liegende gewünschte Winkelwerte einstellbar ist. Diese Maßnahme ermöglicht es, die Vorrichtung an die beim Betrieb zu erwartenden Mengen von Abwasser anzupassen.

In weiterer zweckmäßiger Ausgestaltung der Erfindung erstreckt sich das äußere obere Ende der schrägen Stauwand mindestens bis zum Höhenniveau der oberen Rohrenden der Rohre, und das äußere obere Ende der Stauwand bildet dabei eine weitere Überlaufkante für die Flüssigkeit. Weiterhin ist es vorteilhaft, daß am äußeren oberen Ende der schrägen Stauwand ein Endstück vorgesehen ist, welches in der Höhe verstellbar ist, so daß die durch das Endstück gebildete Überlaufkante auf ein Höhenniveau oberhalb der Überlaufkanten der einzelnen Rohre einstellbar ist. Dadurch läßt sich bei der Inbetriebnahme der Vorrichtung eine Justierung bzw. auch eine Nachjustierung in einfacher Weise erreichen.

Gemäß einer weiteren vorteilhaften Ausgestaltung der Erfindung sind die Rohre in Strömungsrichtung der Flüssigkeit gesehen in mehreren Reihen hintereinander angeordnet, wobei die nebeneinander liegenden Rohre schräg zueinander versetzt angeordnet sind.

Die sich nach oben erstreckenden Rohre bilden mit ihren Rohrwandungen für die strömende Flüssigkeit eine Art Stauwand, deren Effekt allerdings dadurch eingeschränkt ist, daß die Rohre rund ausgebildet sind. Durch die versetzte Anordnung ist gewährleistet, daß sich der durch die Rohre hervorgerufene Rückstau auf die strömende Flüssigkeit soweit in Grenzen gehalten ist, daß sich der Rückstau nicht in nachteiliger Weise auf die angestrebte gleichmäßige Strömungsgeschwindigkeit der Flüssigkeit in der Bestrahlungskammer auswirkt.

Weitere vorteilhafte Ausgestaltungen der Erfindung ergeben sich aus den Unteransprüchen und aus der Zeichnung.

Anhand des in der Zeichnung dargestellten Ausführungsbeispiels wird die Erfindung nachfolgend näher erläutert. Es zeigen:
- Fig. 1: eine schematische Seitenansicht einer Vorrichtung zur UV-Bestrahlung von Flüssigkeiten mit einer Auslaufkammer,
- Fig. 2: eine Draufsicht auf die Stauwand gemäß Fig. 1, und
- Fig. 3: eine Seitenansicht der Stauwand gemäß Fig. 2.

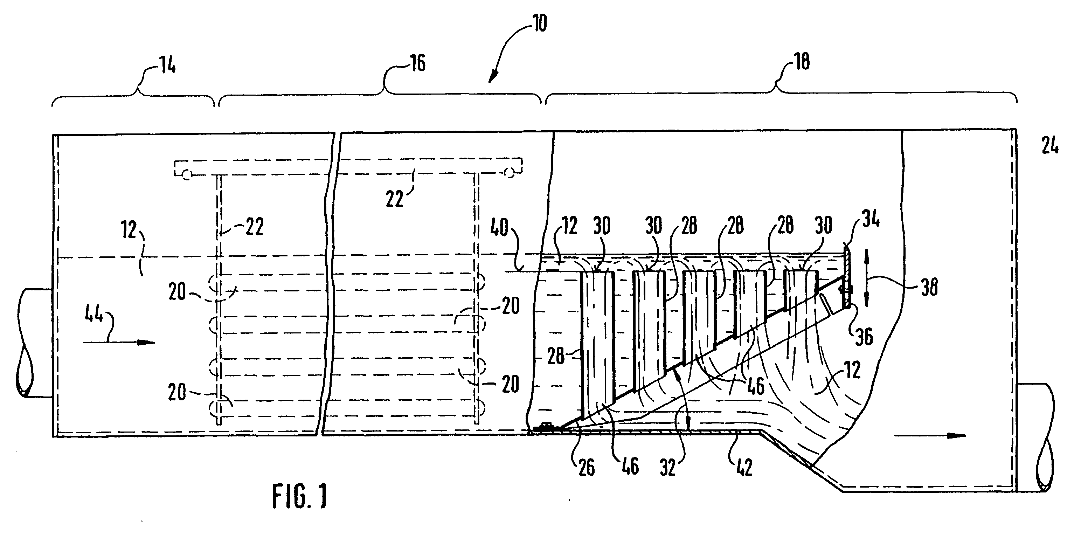

Die Darstellung gemäß Fig. 1 zeigt eine erfindungsgemäße Vorrichtung 10 zur UV-Bestrahlung von vorgereinigtem Abwasser 12, welches von einer nicht dargestellten Kläranlage kommt. Die Vorrichtung 10 ist als ein nach oben offenes Gerinne 24 mit einem Gerinneboden 42 und zwei sich senkrecht erstreckenden Seitenwänden ausgebildet.

Die Vorrichtung 10 umfaßt eine Einlaufkammer 14, über welche das Abwasser 12 der Vorrichtung 10 zugeführt wird, und an die Einlaufkammer 14 schließt sich eine Bestrahlungskammer 16 an. Auf die Bestrahlungskammer 16 folgt eine Auslaufkammer 18, von welcher das Abwasser 12 abgeleitet und dem natürlichen Wasserkreislauf zugeführt wird.

In der Bestrahlungskammer 16 befinden sich nebeneinander und übereinander im Abstand angeordnete UV-Strahlungsquellen 20, die sich in der Strömungsrichtung 44 des Abwassers 12 erstrecken. Die UV-Strahlungsquellen 20 sind in einem Rahmengestell 22 angeordnet, so daß die UV-Strahlungsquellen von oben her in das offene Gerinne 24 eingesetzt werden können. Die UV-Strahlungsquellen 20 können auch senkrecht zur Strömungsrichtung 44 des Abwassers angeordnet sein.

In der Auslaufkammer 18 befindet sich unter einem flachen Winkel 32 eine schräg verlaufende Stauwand 26, welche praktisch am Anfang der Auslaufkammer 18 eine schräg nach oben hochgezogene Verlängerung des Gerinnebodens 42 bildet.

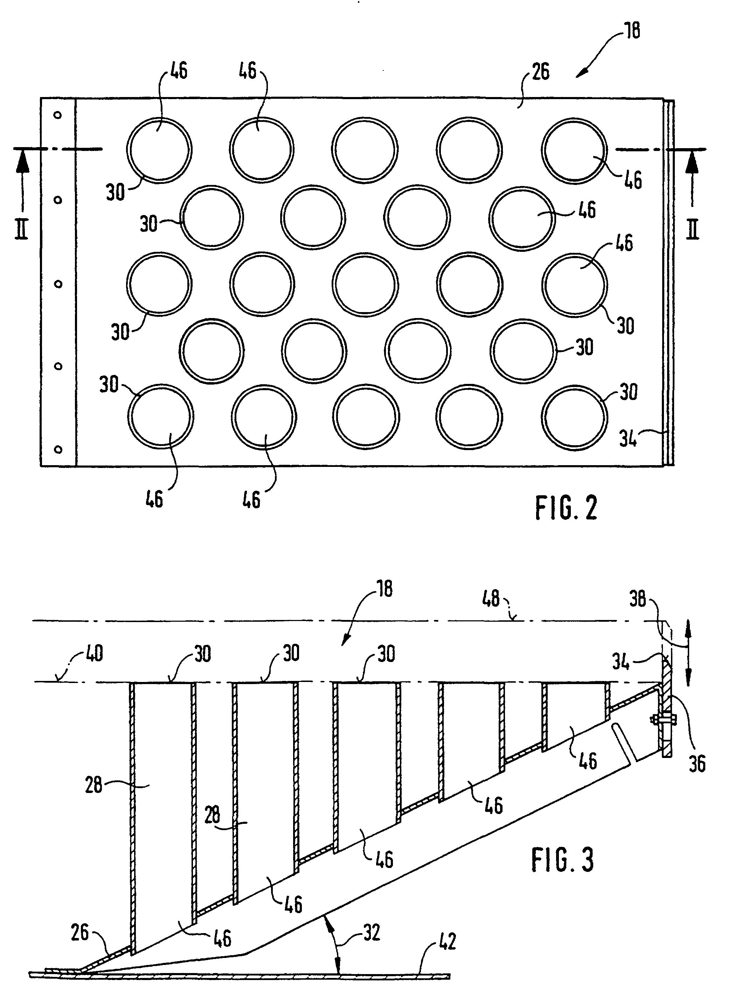

Wie auch in der Draufsicht gemäß Fig. 2 und der Seitenansicht gemäß Fig. 3 zu erkennen ist, befinden sich auf der schrägen Stauwand 26 senkrecht angeordnete Rohre 28, die jeweils an ihren beiden Enden offen sind. Die oberen Enden der Rohre 28 bilden Überlaufkanten für das Abwasser 12. Die schräge Stauwand 26 ist an denjenigen Stellen, an denen die Rohre 28 auf der Stauwand 26 befestigt sind, mit den Rohrquerschnitten entsprechenden Öffnungen 26 versehen. Das über die Überlaufkanten 30 der Rohre 28 fließende Abwasser 12 kann somit durch die unteren Öffnungen der Rohre bzw. durch die unteren Öffnungen 46 der schrägen Stauwand 26 abfließen und dem Ableitungssystem bzw. dem natürlichen Wasserkreislauf zugeführt werden.

Bei der erfindungsgemäßen Vorrichtung wird also auf eine senkrecht zur Strömungsrichtung 44 des Abwassers 12 verlaufende Stauwand verzichtet. Vielmehr verläuft die Stauwand 26 unter einem flachen Winkel schräg und leicht ansteigend, so daß das in die Auslaufkammer 18 gelangende Abwasser nicht abrupt und plötzlich, sondern nur allmählich abgebremst wird. Dadurch läßt sich ein Rückstau des Abwassers 12 verhindern, so daß das Strömungsprofil in der Bestrahlungskammer 16 in gewünschter Weise gar nicht bzw. nur unmerklich beeinflußt wird.

Die Vorrichtung 10 arbeitet auch dann zufriedenstellend, wenn größere Mengen von Abwasser 12 anfallen. Durch die Vielzahl der Rohre 28 ist nämlich gewährleistet, daß auch große Mengen von Abwasser 12 ungehindert und ohne große Wasserstands-Schwankungen aus der Bestrahlungskammer 16 abfließen können.

Das äußere obere Ende der schrägen Stauwand 26, die sich über die gesamte Breite des Gerinnes 24 erstreckt, reicht mindestens bis zum Höheniveau 40 der oberen Rohrenden der Rohre 28. Wie Fig. 1 und Fig. 3 zeigen, befindet sich an den äußeren oberen Ende der schrägen Stauwand 26 ein Endstück 36, und durch den Doppelpfeil 38 ist angedeutet, daß sich dieses senkrecht verlaufende Endstück 36 in senkrechter Richtung zur Strömungsrichtung 44 verstellen läßt. Die obere Seite des Endstückes 36 bildet für das Abwasser 12 eine weitere Überlaufkante 34. Durch Verstellen des Endstückes 36 in senkrechter Richtung läßt sich der Pegel des Abwassers 12 in der Auslaufkammer 18 beeinflussen, und in Fig. 3 ist dargestellt, daß eine Pegeleinstellung auf ein Niveau 48 möglich ist.

In den Darstellungen gemäß Fig. 1 - 3 sind die Rohre 28 senkrecht zur Strömungsrichtung 44 angeordnet. Es ist im Rahmen der Erfindung aber auch möglich, die Rohre abweichend von der senkrechten Schräge anzuordnen, ohne daß dadurch die Wirkungsweise der erfindungsgemäßen Vorrichtung 10 beeinflußt wird. In jedem Fall verlaufen die Überlaufkanten 30 der Rohre 28 parallel zum Gerinneboden 42.

Normalerweise besitzen die Rohre 28 einen kreisförmigen Querschnitt, jedoch ist es auch möglich, daß die Rohre eine andere Form besitzen, zum Beispiel elliptisch ausgebildet werden.

Als zweckmäßig für eine optimale Arbeitsweise der erfindungsgemäßen Vorrichtung 10 hat sich erwiesen, wenn die Rohre 28 gleich ausgebildet sind, natürlich mit Ausnahme ihrer Rohrlänge, die wegen der schrägen Stauwand 26 unterschiedlich ist, damit die Überlaufkanten 30 auf einem gleichen Niveau liegen. Der kleinste Abstand der Rohre 28 zueinander entspricht mindestens dem halben Innenradius der Rohre. Mit einer solchen Anordnung arbeitet die erfindungsgemäße Vorrichtung unter optimalen Bedingungen.

Im Rahmen der Erfindung kann es auch von Vorteil sein, die oberen Enden der Rohre 28 trichterförmig auszubilden. Dadurch vergrößert sich am oberen Trichterende der Umfang der Rohre, so daß insgesamt die zur Verfügung stehende Länge von den Überlaufkanten 30 erhöht wird und mehr Abwasser 12 durch die Rohre 28 abfließen kann.

## Patentansprüche

1. Vorrichtung (10) zur UV-Bestrahlung von Flüssigkeiten (12), insbesondere von vorgereinigtem Abwasser, mit einer Bestrahlungskammer (16), durch welche die Flüssigkeit (12) fließt und in welcher UV-Strahlungsquellen (20) angeordnet sind, und mit einer sich in Strömungsrichtung (44) der Flüssigkeit (12) an die Bestrahlungskammer (16) anschließenden und zum Abfluß der Flüssigkeit (12) vorgesehenen Auslaufkammer (18), die als ein oben offenes Gerinne (24 )mit einem unteren Gerinneboden (42) und zwei Seitenwänden ausgebildet ist, und die mindestens einen Wehrkörper besitzt, der eine die Strömung der Flüssigkeit (12) abbremsende und den hinteren Abschluß der Auslaufkammer (18) bildende Stauwand mit oberen Überlaufkanten aufweist, über welche die aufgestaute Flüssigkeit (12) überläuft und abfließt, **dadurch gekennzeichnet, daß** sich die Stauwand (26) in Strömungsrichtung (44) als nach oben hochgezogene Verlängerung des Gerinnebodens (42) schräg vom vorderen Ende zum hinteren Ende der Auslaufkammer (18) in einem flachen Winkel innerhalb eines Winkelbereiches zwischen 10° und kleiner als 60° zum Gerinneboden (42) erstreckt, daß auf der schrägen Stauwand (26) mehrere voneinander getrennte einzelne Wehrkörper angeordnet sind, daß die Wehrkörper durch sich nach oben erstreckende hohle und an beiden Enden offene Rohre (28) gebildet sind, deren oberen Enden Überlaufkanten (30) bilden, und daß die Stauwand (26) an den Stellen, an denen die Rohre (28) auf der Stauwand (26) befestigt sind, mit den Rohrquerschnitten entsprechenden Öffnungen (46) zum Abfluß der über die Überlaufkanten (30) der Rohre (28) fließenden Flüssigkeit (12) versehen ist, und daß die oberen Enden der einzelnen Rohre (28) auf einem gleichen Höhenniveau (40) liegen und daß die Rohre (28) senkrecht oder schräg zur Strömungsrichtung (44) angeordnet sind, und daß ihre Überlaufkanten (30) parallel zum Gerinneboden (42) verlaufen.

2. Vorrichtung nach Anspruch 1, **dadurch gekennzeichnet, daß** die schräge Stauwand (26) innerhalb des Winkelbereiches zwischen 10° und 50° verschwenkbar und auf gewünschte Winkelwerte einstellbar ist.

3. Vorrichtung nach einem der vorhergehenden Ansprüche 1 - 2, **dadurch gekennzeichnet, daß** das äußere obere Ende der schrägen Stauwand (26) sich bis zum Höhenniveau (40) der oberen Rohrenden der Rohre (28) erstreckt und eine weitere Überlaufkante (34) für die Flüssigkeit (12) bildet.

4. Vorrichtung nach Anspruch 3, **dadurch gekennzeichnet, daß** am äußeren oberen Ende der schrägen Stauwand (26) ein Endstück (36) vorgesehen ist, welches in der Höhe verstellbar (38) ist, so daß die durch das Endstück (36) gebildete Überlaufkante (34) auf ein Höhenniveau sowohl oberhalb als auch unterhalb der Überlaufkanten (34) der einzelnen Rohre (28) einstellbar ist.

5. Vorrichtung nach Anspruch 1, **dadurch gekennzeichnet, daß** die Rohre (28) in Strömungsrichtung (44) der Flüssigkeit (12) gesehen in mehreren Reihen hintereinander angeordnet sind, und daß die nebeneinander liegenden Reihen schräg zueinander versetzt angeordnet sind.

6. Vorrichtung nach Anspruch 1, **dadurch gekennzeichnet, daß** die Rohre (28) einen kreisförmigen Querschnitt besitzen.

7. Vorrichtung nach Anspruch 1, **dadurch gekennzeichnet, daß** die senkrecht angeordneten Rohre (28) einen von der Kreisform abweichenden Querschnitt besitzen.

8. Vorrichtung nach einem der vorhergehenden Ansprüche 1 - 7, **dadurch gekennzeichnet, daß** die Rohre (28) mit Ausnahme ihrer Rohrlänge gleich ausgebildet sind, und daß der kleinste Abstand der Rohre zueinander mindestens ihrem halben Innenradius entspricht.

9. Vorrichtung nach einem der vorhergehenden Ansprüche 1 - 8, **dadurch gekennzeichnet, daß** die oberen Enden der einzelnen Rohre (28) trichterförmig ausgebildet sind.

## Revendications

1. Dispositif (10) à rayons ultraviolets pour le traitement de liquides (12), en particulier d'eaux résiduaires prétraitées, avec une chambre d'exposition (16) traversée par le liquide (12) et dans laquelle des sources de rayons ultraviolets (20) sont disposées, et avec une chambre de décharge (18), adjacente à la chambre d'exposition (16) dans la direction d'écoulement (44) du liquide (12) et prévue pour la décharge du liquide (12), qui est conçue comme conduit (24) ouvert vers le haut avec un fond de conduit inférieur (42) et deux parois latérales, et qui possède au moins un corps de barrage présentant une paroi de barrage freinant le courant de liquide (12) et formant l'extrémité arrière de la chambre de décharge (18) avec des bords-déversoirs supérieurs, par-dessus lesquels le liquide (12) retenu se déverse et se décharge, **caractérisé en ce que** la paroi de barrage (26) s'étend dans la direction d'écoulement (44), comme un prolongement vers le haut du fond de conduit (42), diagonalement de l'extrémité avant vers l'extrémité arrière de la chambre de décharge (18), en formant un angle plan, à l'intérieur d'une plage d'angle supérieure à 10° et inférieure à 60°, avec le fond de conduit (42), **en ce que** plusieurs corps de barrage individuels séparés les uns des autres sont disposés sur la paroi de barrage inclinée, **en ce que** les corps de barrage sont formés par des tubes (28) creux et ouverts aux deux extrémités qui s'étendent vers le haut, dont les extrémités supérieures forment des bords-déversoirs (30) et **en ce qu'**aux endroits auxquels les tubes (28) sont fixés sur la paroi de barrage (26), celle-ci est pourvue d'ouvertures (46) correspondant aux sections transversales des tubes pour l'écoulement du liquide s'écoulant par dessus les bords-déversoirs (30) des tubes (28), **en ce que** les extrémités supérieures des différents tubes (28) se trouvent sur un même niveau de hauteur (40) et **en ce que** les tubes (28) sont disposés perpendiculairement ou diagonalement à la direction d'écoulement (44) et que leurs bords-déversoirs (30) s'étendent parallèlement au fond du conduit (42).

2. Dispositif selon la revendication 1, **caractérisé en ce que** la paroi de barrage inclinée (26) est orientable à l'intérieur d'une plage d'angle supérieure à 10° et inférieure à 50° et réglable de manière à correspondre à des valeurs d'angle souhaitées.

3. Dispositif selon l'une des revendications précédentes 1 à 2, **caractérisé en ce que** l'extrémité supérieure extérieure de la paroi de barrage inclinée (26) s'étend jusqu'au niveau de hauteur (40) des extrémités supérieures des tubes (28) et forme un bord-déversoir supplémentaire (34) pour le liquide (12).

4. Dispositif suivant la revendication 3, **caractérisé en ce qu**'à l'extrémité supérieure extérieure de la paroi de barrage inclinée (26) est prévu un embout (36) qui est réglable (38) en hauteur, de manière que le bord-déversoir (34) formé par l'embout (36) peut être réglé sur un niveau de hauteur aussi bien supérieur qu'inférieur aux bords-déversoirs (34) des différents tubes (28).

5. Dispositif selon la revendication 1, **caractérisé en ce que** les tubes (28) sont disposés en plusieurs rangs les uns derrière les autres, vus dans la direction d'écoulement (44) du liquide (12) et que les rangs se trouvant côte à côte sont disposés diagonalement décalés les uns par rapport aux autres.

6. Dispositif selon la revendication 1, **caractérisé en ce que** les tubes (28) possèdent une section transversale circulaire.

7. Dispositif selon la revendication 1, **caractérisé en ce que** les tubes (28) disposés verticalement possèdent une section transversale qui s'écarte de la forme circulaire.

8. Dispositif selon l'une des revendications précédentes 1 à 7, **caractérisé en ce que** les tubes (28) sont de conception identique, à l'exception de la longueur de tube, et que l'écart le plus petit entre les tubes correspond au moins à la moitié de leur rayon intérieur.

9. Dispositif selon l'une des revendications précédentes 1 à 8, **caractérisé en ce que** les extrémités supérieures des différents tubes (28) sont conçues en forme d'entonnoir.

## Claims

1. Apparatus (10) for the UV irradiation of liquids (12), particularly precleaned waste water, having an irradiation chamber (16) through which the liquid (12) flows and in which are located UV radiation sources (20), and with a discharge chamber (18) provided in the flow direction (44) of the liquid (12), which is connected to the irradiation chamber (16) and which discharges the liquid (12), said chamber (18) being constructed as a top-open channel (24) with a lower channel bottom (42) and two side walls and which has at least one weir, which has a baffle plate with upper overflow edges decelerating the flow of the liquid (12) and forming the rear termination of the discharge chamber (18) and over which the retained liquid (12) flows and is discharged, **characterized in that** the baffle plate (26) extends in the flow direction (44) as an upwardly raised extension of the channel bottom (42) in inclined manner from the front end to the rear end of the discharge chamber (18) and with a shallow angle within the range 10° and smaller than 60° to the channel bottom (42), that on the sloping baffle plate (26) are provided several separate, individual weirs, that the weirs are formed by upwardly extending, hollow pipes (28) which are open at both ends and whose upper ends form overflow edges (30) and that at the locations where the pipes (28) are fixed to the baffle plate (26), the latter is provided with openings (46) corresponding to the pipe cross-sections for the discharge of the liquid (12) flowing over the overflow edges (30) of the pipes (28) and that the upper ends of the individual pipes (28) are at the same height level (40) and that the pipes (28) are positioned vertically or in inclined manner to the flow direction (44) and their overflow edges (30) are parallel to the channel bottom (42).

2. Apparatus according to claim 1, **characterized in that**, within the angular range between 10° and 50°, the sloping baffle plate (26) can be pivoted and adjusted to the desired angular values.

3. Apparatus according to one of the preceding claims 1 and 2, **characterized in that** the outer, upper end of the sloping baffle plate (26) extends up to the height level (40) of the upper ends of the pipes (28) and forms a further overflow edge (34) for the liquid (12).

4. Apparatus according to claim 3, **characterized in that** at the upper, outer end of the sloping baffle plate (26) is provided an end section (36), whose height can be adjusted (38), so that the overflow edge (34) formed by the end section (36) is adjustable to a height level both above and below the overflow edges (34) of the individual pipes (28).

5. Apparatus according to claim 1, **characterized in that**, considered in the flow direction (44) of the liquid (12), the pipes (28) are arranged successively in several rows and that the juxtaposed rows are offset in sloping manner to one another.

6. Apparatus according to claim 1, **characterized in that** the pipes (28) have a circular cross-section.

7. Apparatus according to claim 1, **characterized in that** the vertically positioned pipes (28) have a non-circular cross-section.

8. Apparatus according to one of the preceding claims 1 to 7, **characterized in that**, with the exception of their length, the pipes (28) are constructed identically and that the smallest mutual spacing of the pipes corresponds to at least half the inside radius thereof.

9. Apparatus according to one of the preceding claims 1 to 8, **characterized in that** the upper ends of the individual pipes (28) are funnel-shaped.
